# FASCICULE DE BREVET EUROPEEN

(11) **EP 1 335 754 B1**
(45) Date de publication et mention de la délivrance du brevet: **20.02.2008**
(21) Numéro de dépôt: 01996397.4
(22) Date de dépôt: 16.11.2001
(51) Int. Cl.: A61L 2/26

(54) **OPERCULE POUR EMBALLAGE POUR PRODUITS A STERILISER A L'AIDE D'UN FLUIDE STERILISANT A HAUTE TEMPERATURE**
VERSCHLUSSMEMBRAN FÜR EINE VERPACKUNG FÜR MIT EINEM HOCHTEMPERATURSTERILISIERUNGSFLUID ZU STERILISIERENDE PRODUKTE
CLOSURE FOR A PACKAGE FOR PRODUCTS TO BE STERILISED WITH A HIGH-TEMPERATURE STERILISING FLUID

(30) Priorité: 20.11.2000 FR 0014976
(43) Date de publication de la demande: 20.08.2003
(73) Titulaire: Becton Dickinson France, 38800 Le Pont de Claix (FR)
(72) Inventeur: GRIMARD, Jean-Pierre, F-38450 VIF (FR); THIBAULT, Jean-Claude, F-38120 SAINT-EGREVE (FR)
(74) Mandataire: Brédeville, Odile Marie
(86) Numéro de dépôt international: PCT/FR2001/003614
(87) Numéro de publication internationale: WO 2002/040065

(56) Documents cités:
- EP-A- 0 266 688
- EP-A- 0 846 445
- WO-A-91/11204
- WO-A-91/11374
- DE-A- 2 839 219

## Description

La présente invention se rapporte au domaine des emballages stériles ou stérilisés, et plus particulièrement aux emballages destinés à transporter des produits stérilisés ou destinés à être stérilisés.

Les conditions de stérilité dans lesquelles doivent se dérouler certaines étapes de manipulation ou de transport des produits ou ustensiles destinés à un usage médical sont très contraignantes et en particulier dans l'industrie pharmaceutique. Il est donc de grande importance de réaliser des emballages compatibles avec de telles exigences.

Dans la suite de la description, il sera fait mention d'un certain nombre d'expressions qu'il convient de définir ci-après.

Par l'expression "matériau ou feuille sélectivement étanche", telle qu'utilisée dans la présente description ainsi que dans les revendications, on entend que le matériau est conçu en termes de structure de manière à contrôler tout échange de l'intérieur de l'emballage avec son environnement extérieur. Ceci signifie entre autres que l'emballage est étanche individuellement ou en combinaison à la contamination par des microorganismes, bactéries et/ou un matériau biologiquement actif, susceptible de venir en contact avec l'emballage lors de sa manipulation, tout en restant perméable à un gaz de stérilisation, par exemple de l'oxyde d'éthylène ou un fluide stérilisant thermique à haute température, par exemple de la vapeur d'eau.

Par "matériau plastique", il faut entendre tout matériau choisi dans les familles des polymères tels que les styréniques, acryliques, polysulfones, polycarbonates, polyesters, polyoléfines, etc...., en incluant les copolymères, combinaisons et alliages de polymères.

Par "haute température", il faut entendre des températures proches des températures de déformation des matériaux plastiques en présence.

Il sera également fait mention d'un écran vis-à-vis d'une irradiation électronique, lequel doit être compris comme un matériau susceptible d'absorber l'énergie cinétique des électrons issus d'un faisceau et par conséquent de ralentir, voire d'empêcher ces derniers de traverser ledit matériau, ou de réfléchir lesdits électrons.

L'expression "écran vis-à-vis d'un rayonnement lumineux" doit être comprise comme désignant un matériau permettant d'empêcher à un rayonnement lumineux, par exemple pulsé ou ultraviolet, de le traverser.

Les termes "transparent" et "opaque" sont à considérer vis-à-vis d'une irradiation électronique d'une part et vis-à-vis d'un rayonnement lumineux d'autre part. Un matériau est transparent s'il est susceptible d'être traversé par un rayonnement lumineux ou par des électrons lorsqu'il est soumis à une irradiation électronique. Un matériau est par conséquent opaque vis-à-vis d'une irradiation électronique ou d'un rayonnement lumineux s'il permet d'absorber l'énergie cinétique des électrons ou de les réfléchir, ou s'il empêche un rayonnement lumineux de le traverser. L'opacité et/ou la transparence d'un matériau sont le plus souvent déterminés à partir d'un couple de paramètres, à savoir l'épaisseur et la densité et le cas échéant à partir du coefficient de réflexion vis-à-vis des électrons.

On connaît des emballages pour produits stériles ou destinés à être stérilisés par un gaz du type oxyde d'éthylène, comportant une boîte en matière plastique et un couvercle réalisé en matériau sélectivement étanche, permettant de sceller ladite boîte.

Certains emballages comme ceux utilisés pour transporter des seringues avant leur remplissage par un produit actif ou un médicament, sont actuellement transportés dans des boîtiers en plastique, par exemple du polystyrène, recouvertes d'une feuille de couverture réalisée avec un matériau sélectivement étanche. Ce dernier est par exemple une feuille à base de filaments de PEHD (polyéthylène haute densité) ou autre polymère, liés notamment par l'intermédiaire de chaleur et de pression. Un tel produit, considéré comme chimiquement inerte, est par exemple commercialisé sous la marque TYVEK^{®}.

Des produits destinés à être stérilisés sont ainsi disposés à l'intérieur d'une boîte, laquelle est ensuite scellée par l'intermédiaire de la feuille sélectivement étanche. Un fluide de stérilisation pénètre ensuite dans la boîte à travers la feuille de matériau sélectivement étanche. La boîte contenant les produits stérilisés est alors disposée dans un sac de protection pour la transporter. La boîte peut également être disposée dans un tel sac avant l'opération de stérilisation. Dans ce cas, le sac est pourvu d'une fenêtre recouverte d'un matériau perméable à un fluide de stérilisation.

A titre d'exemple, une telle boîte ou emballage peut contenir des seringues destinées à être remplies par un médicament dans une salle stérile ou à environnement contrôlé. Avant le remplissage desdites seringues, il est nécessaire d'ouvrir le sac de protection et de décontaminer l'emballage éventuellement contaminé, avant son introduction par exemple dans une salle stérile. Une telle décontamination peut être obtenue à l'aide d'un faisceau d'électrons développant une énergie suffisante pour présenter après la traversée de la feuille de couverture (matériau sélectivement étanche), une dose d'irradiation de 25 kGy. Cela permet d'assurer que le matériau sélectivement étanche a été décontaminé dans toute son épaisseur, et en particulier dans la zone scellée à l'interface de la boîte et dudit matériau sélectivement étanche.

Ce type de décontamination par l'intermédiaire d'un faisceau d'électrons peut présenter cependant des inconvénients. En effet, avec une décontamination à l'aide d'une irradiation électronique, des électrons traversant la feuille du matériau sélectivement étanche risquent d'une part de modifier ou d'altérer le matériau constitutif des seringues ou produits disposés dans la boite, par exemple du verre, et d'autre part de générer avec l'oxygène de l'air contenu dans ladite boîte, de l'ozone. Ce dernier peut altérer des éléments ou des pièces en caoutchouc comme par exemple des capuchons d'aiguilles montés sur des seringues ou polluer l'atmosphère. Le remplissage des seringues avec un médicament peut également être déconseillé dans un milieu contenant de l'ozone.

L'utilisation d'une décontamination à l'aide d'un rayonnement lumineux n'est par ailleurs pas indiquée car ledit rayonnement lumineux n'est généralement pas en mesure de traverser la feuille de matériau sélectivement étanche et par conséquent, d'atteindre une zone localisée à l'interface du couvercle et de la boîte. Cette zone contenant par exemple une couche de colle peut présenter des irrégularités et il est par conséquent indispensable d'atteindre ces irrégularités éventuellement contaminées, avec un moyen de décontamination.

Il est connu de réaliser un emballage en matériau thermoplastique, contenant une charge stérilisable ou stérilisée par mise en contact avec un fluide stérilisant thermique à haute température, ledit emballage comportant au moins un organe de communication fluidique entre l'intérieur et l'extérieur dudit emballage consistant en au moins un cadre circonscrivant une ouverture et un opercule obturant l'ouverture, et dont la bordure périphérique est continûment liée de manière étanche audit cadre, ledit opercule comprenant, une feuille d'un matériau sélectivement étanche dont le seuil de coupure de l'extérieur vers l'intérieur, arrête toutes particules contaminantes et permet le passage du fluide stérilisant thermique, ledit matériau sélectivement étanche étant toutefois susceptible de se déformer principalement dans le plan de ladite feuille à ladite haute température.

Un tel emballage peut présenter des inconvénients.

En effet, une stérilisation à l'aide d'un fluide stérilisant à haute température, par exemple de la vapeur d'eau, peut provoquer une rétraction du matériau sélectivement étanche, par exemple le matériau appelé TYVEK^{®}, ce qui génère, compte tenu des dimensions relativement importantes des boîtes et emballages, des tensions et des déformations sur le moyen de liaison entre ladite feuille de matériau sélectivement étanche et le cadre. De telles tensions peuvent provoquer une déformation de la boîte et/ou une désolidarisation entre la feuille de matériau sélectivement étanche et ladite boite ou le cadre.

Le document WO 91/11204 A décrit un opercule pour emballage de produits stérilisés comprenant une feuille d'un matériau sélectivement étanche, dont le seuil de coupure de l'extérieur vers l'intérieur de l'emballage arrête les particules contaminantes et permet le passage d'un fluide stérilisant thermique à des températures élevées.

L'objectif de la présente invention est de fournir un emballage pour produits stérilisables à l'aide d'un fluide stérilisant thermique à haute température, ne présentant pas les inconvénients de l'état de la technique, c'est-à-dire pour lequel l'utilisation d'un fluide stérilisant thermique à haute température n'altère pas l'intégrité dudit emballage.

Un autre objectif de l'invention est de fournir un emballage susceptible de subir en plus d'une stérilisation à haute température, une décontamination par exemple à l'aide d'un faisceau d'électrons ou d'un rayonnement lumineux ou ultraviolet.

Selon l'invention, l'emballage comprend un moyen de compensation des contraintes et des déformations de la boîte et du matériau sélectivement étanche, lorsque ledit opercule est en contact avec le fluide stérilisant thermique, ledit moyen de compensation étant déterminé pour relaxer au moins en partie la contrainte dans la direction de l'ouverture de l'organe de communication fluidique appliquée au cadre du fait desdites déformations.

Selon un mode de réalisation de l'emballage conforme à l'invention, le matériau sélectivement étanche présente une zone de liaison périphérique sur laquelle est répartie de façon continue, une colle compatible avec une stérilisation à haute température et une zone centrale restant dépourvue de colle. La colle peut également être répartie de façon discrète (points de colle successifs) et être transformée en une répartition continue après l'opération de scellage.

Selon un mode de réalisation de l'emballage conforme à l'invention, le moyen de compensation est du type actif et consiste en une conformation en relief, de la feuille du matériau sélectivement étanche, dont la surface développée excède la surface apparente de ladite feuille au point d'annuler, lors de son dépliement, la contrainte omnidirectionnelle du fait de la rétraction planaire de ladite feuille.

Selon un autre mode de réalisation conforme à l'invention, l'opercule comprend un liséré d'un matériau étanche vis-à-vis du fluide stérilisant thermique, dont la bordure extérieure est continûment liée de manière étanche au cadre de l'organe de communication fluidique, et dont la bordure intérieure est continûment liée de manière étanche à la feuille du matériau sélectivement étanche, ledit liséré comprenant ledit moyen de compensation.

Selon un autre mode de réalisation conforme à l'invention, le moyen de compensation est du type passif, et est susceptible d'être obtenu par un prétraitement thermique du matériau sélectivement étanche, le déformant définitivement de manière planaire.

D'autres caractéristiques et avantages ressortiront également de la description détaillée figurant ci-après, donnée à titre d'exemple, par référence au dessin annexé, dans lequel :
- la figure 1 représente, en coupe, un exemple de réalisation d'un emballage conforme à l'invention ;
- la figure 2 représente, en coupe, un autre exemple de réalisation d'un emballage conforme à l'invention ;
- la figure 3 représente, en coupe, un exemple complémentaire de réalisation d'un emballage conforme à l'invention ;
- la figure 4 représente, en coupe, une variante de réalisation de l'emballage de la figure 1.

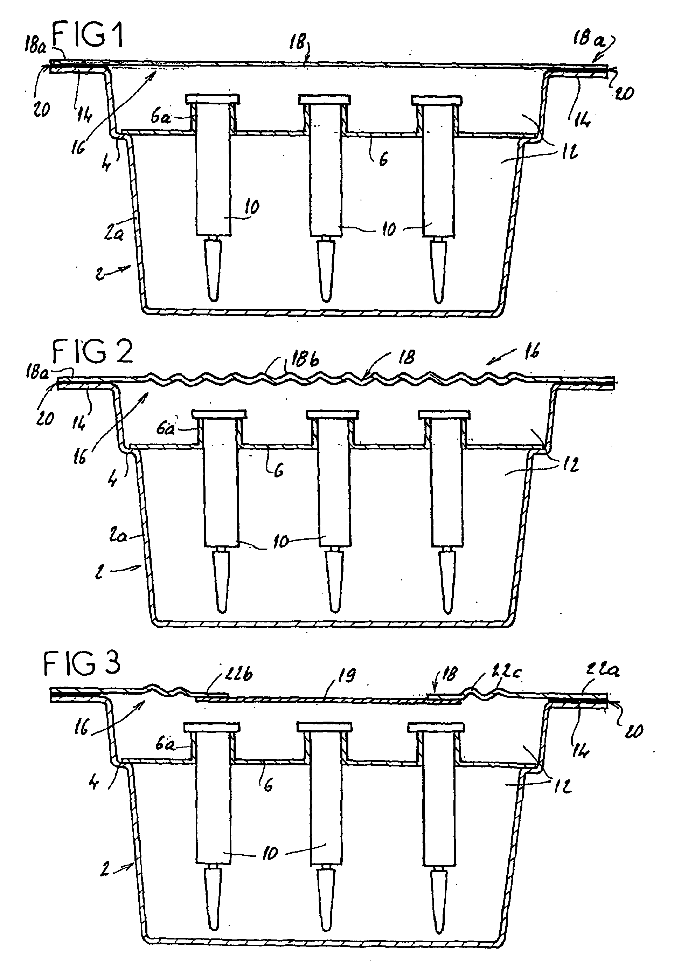

L'emballage conforme à l'invention représenté à la figure 1 comprend un matériau plastique réalisé sous forme de boîte 2 généralement rigide dans lequel est disposée une charge stérilisable ou stérilisée. Le boîtier 2 présente un bord périphérique interne 4 sur lequel repose un support 6 présentant des cheminées 6a dans lesquelles sont engagées, par exemple des seringues 10 constituant la charge stérilisable ou stérilisée. La boîte 2 délimite ainsi un volume interne 12. Il présente également un bord périphérique supérieur 14 s'étendant sensiblement horizontalement vers l'extérieur. L'emballage peut ainsi recevoir au moins un organe de communication fluidique 16 entre l'intérieur et l'extérieur dudit emballage.

L'organe de communication fluidique 16 consiste en au moins un cadre circonscrivant une ouverture et un opercule 18 obturant ladite ouverture. Le cadre par exemple rigide ou semi-rigide est par exemple constitué par le bord périphérique supérieur 14. L'opercule 18 présente un bord périphérique 18a continûment lié, de manière étanche, audit cadre, et en l'occurrence au bord périphérique supérieur 14. Cette liaison est par exemple obtenue à l'aide d'une couche de colle 20. L'opercule 18 comprend au moins une feuille d'un matériau sélectivement étanche dont le seuil de coupure de l'extérieur vers l'intérieur de l'emballage, arrête toutes particules contaminantes et permet le passage du fluide d'un stérilisant thermique.

Le matériau sélectivement étanche comprend par exemple une feuille d'un matériau appelé TYVEK^{®}. Ce dernier est susceptible de se contracter entre autres dans le plan de ladite feuille à une haute température correspondant à une température de stérilisation. Cette température est par exemple de 121°C à 130° C, lorsque le fluide stérilisant est par exemple de la vapeur d'eau. Le fluide stérilisant thermique à haute température pénètre donc dans le volume interne 12 en traversant l'opercule 18. La colle 20 sera choisie de préférence parmi les types sans solvant, par exemple à base acqueuse ou thermofusibles (hot melt)

La colle 20 sera de préférence "pelable", c'est-à-dire sans génération de particules ou fibres lors du décollement de l'opercule 18 pour l'ouverture de la boîte 2.

La colle 20 est choisie de telle façon que son éventuel point de ramollissement sera supérieur à la plus haute température atteinte durant les opérations de stérilisation.

La colle 20 permet de réaliser une liaison entre la bordure périphérique 18a de l'opercule 18 et le cadre de l'organe de communication fluidique 16. Cette liaison est mécaniquement résistante vis-à-vis de la contrainte omnidirectionnelle du fait principalement de la déformation planaire de la feuille de matériau sélectivement étanche constituant l'opercule 18.

Selon un exemple de réalisation, la boîte 2 présente une paroi 2a mécaniquement résistante vis-à-vis de la contrainte liée à la rétraction planaire de la feuille de matériau sélectivement étanche constituant l'opercule 18.

L'emballage conforme à l'invention comprend ainsi intrinsèquement un moyen de compensation de la rétraction planaire de la feuille de matériau sélectivement étanche lorsque ledit opercule 18 est en contact avec le fluide d'un stérilisant thermique. Le moyen de compensation permet de relaxer au moins en partie la contrainte dans la direction de l'ouverture de l'organe de communication fluidique 16, appliquée au cadre du fait de la rétraction planaire.

Selon le mode de réalisation schématisé par la figure 1, le moyen de compensation du type passif, est susceptible d'être obtenu par un prétraitement thermique du matériau sélectivement étanche en le contractant préalablement de manière planaire. Le matériau sélectivement étanche est ainsi soumis à une haute température, par exemple en utilisant un fluide à haute température, par exemple de la vapeur d'eau traversant ledit matériau sélectivement étanche. On obtient ainsi une rétraction irréversible de sorte qu'une exposition ultérieure à une température élevée (par exemple avec la traversée d'un fluide stérilisant à haute température) évite ou minimise la rétraction complémentaire. Le matériau sélectivement étanche, préalablement rétracté conserve ainsi ses dimensions, et aucune contrainte supplémentaire préjudiciable n'apparaît sur la liaison périphérique réalisée entre la bordure périphérique 18a et le bord supérieur 14,

Selon un autre mode de réalisation de l'emballage conforme à l'invention, le moyen de compensation est du type actif (cf. figures 2 et 3).

L'exemple de réalisation représenté à la figure 2 montre le moyen de compensation consistant en une conformation en relief de la feuille de matériau sélectivement étanche constituant l'opercule 18. La surface développée de cette feuille de matériau sélectivement étanche excède la surface apparente de ladite feuille au point d'annuler, lors de sa déformation ou rétraction, la contrainte liée à la rétraction planaire de ladite feuille. La conformation en relief 18b est du type plissage, repliage, bosselure, ondulation ou gaufrage. Cette conformation en relief permet également d'augmenter la surface d'échange (passage du fluide stérilisant) du matériau sélectivement étanche.

Dans l'exemple représenté à la figure 3, l'opercule 18 comprend un liseré 22 d'un matériau étanche éventuellement vis-à-vis du fluide stérilisant thermique, dont la bordure extérieure 22a est continûment liée de manière étanche au cadre de l'organe de communication fluidique (bord périphérique supérieur 14). La bordure intérieure 22b du liseré 22 est continûment liée de manière étanche à la feuille centrale 19 de matériau sélectivement étanche. La liaison entre la feuille centrale 19 de matériau sélectivement étanche et le bord intérieur 22b du liseré 22 est obtenue par tout moyen et notamment par collage. Dans cet exemple, le liseré 22 comprend le moyen de compensation. Ce dernier consiste par exemple en une autre conformation en relief 22c, par exemple repliée, ondulée ou plissée sur elle-même autour d'une ou plusieurs lignes continues circonscrivant la feuille centrale 19 en matériau sélectivement étanche. La surface développée de ce liseré 22 excède la surface apparente dudit liseré 22 au point d'annuler lors de sa déformation, la contrainte liée à la rétraction isotrope planaire de la feuille centrale 19 de matériau sélectivement étanche.

Selon un exemple de réalisation conforme à l'invention, le matériau constitutif du liseré 22 est transparent vis-à-vis d'un rayonnement ultraviolet stérilisant ou décontaminant.

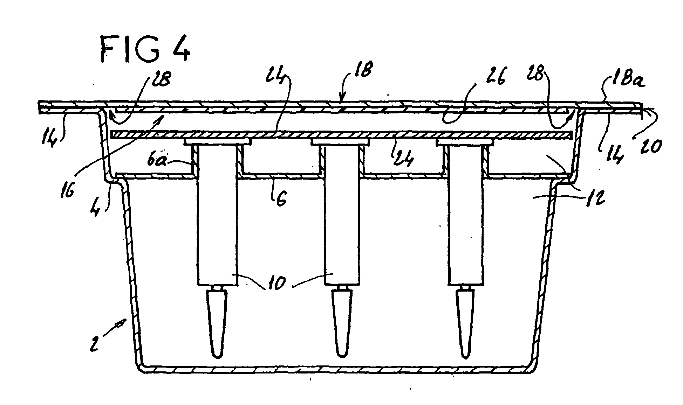

Le matériau sélectivement étanche est par exemple une nappe non tissée de fibres ou filaments d'une matière thermoplastique, par exemple PEHD ou autre polymère, enchevêtrés et liés les uns aux autres par exemple par thermofusion. Le matériau sélectivement étanche peut également être constitué d'un matériau à base de fibres naturelles ou végétales, du type papier, traité ou préparé de façon spécifique, le cas échéant. L'emballage conforme à l'invention peut également comporter au moins une feuille supplémentaire 24 de matériau sélectivement étanche (cf. figure 4) reposant sur les seringues 10 et servant de protection mécanique et/ou d'écran complémentaire pour lesdites seringues 10. Cette feuille complémentaire de matériau sélectivement étanche 24 peut également être disposée dans les exemples de réalisation représentés aux figures 1 à 3.

L'opercule 18 comprend par exemple une première feuille de matériau sélectivement étanche (cf. figure 4) monté sur la boîte 2 de la même manière que décrit pour l'exemple de la figure 1. Cette feuille de matériau sélectivement étanche est avantageusement associée à un écran 26 vis-à-vis d'une irradiation électronique et/ou d'un rayonnement lumineux, visible et/ou par exemple ultraviolet. L'écran 26 peut être constitué d'une ou plusieurs couches ou feuilles de matériau sélectivement étanche supplémentaire, et par conséquent perméables à un fluide stérilisant thermique à haute température.

Cet écran 26 peut être rapporté sur l'opercule 18 par tout moyen connu, et notamment par collage, ou peut être simplement déposé sur les produits 10 à l'intérieur de la boîte 2 préalablement au scellage de celle-ci.

L'écran 26 peut également être réalisé avec un matériau étanche. Dans un tel cas, les dimensions de l'écran 26 sont choisies de manière à ménager une zone 28 périphérique, ou autres interruptions ou orifices, de la feuille de l'opercule 18, en communication directe avec le volume intérieur 12. Le fluide stérilisant thermique à haute température pénètre ainsi dans le volume intérieur 12 en traversant cette zone 28 de la feuille de matériau sélectivement étanche. L'écran 26 est par exemple constitué d'une feuille aluminium.

Selon l'invention, on obtient ainsi un opercule 18 pour emballage rigide de produit stérilisé ou destiné à être stérilisé comprenant au moins une feuille d'un matériau sélectivement étanche, dont le seuil de coupure de l'extérieur vers l'intérieur de l'emballage arrête toutes les particules contaminantes et permet le passage d'un fluide stérilisant thermique à haute température. L'opercule 18 comprend également le moyen de compensation de la déformation de la feuille de matériau sélectivement étanche lors du contact avec le fluide stérilisant thermique à haute température. Un tel opercule 18 conforme à l'invention peut ainsi être fabriqué séparément et rapporté sur tout type d'emballage, de préférence rigide.

D'autres configurations ou réalisations comprenant un écran 26 (non représentées) peuvent également être envisagées sans sortir du cadre de la présente invention. Le matériau sélectivement étanche peut également être constitué à partir de fibres naturelles telles que des fibres de cellulose compatibles avec une stérilisation à la vapeur d'eau, ou encore d'un film microperforé.

Un avantage considérable de l'emballage conforme à l'invention réside dans sa simplicité et dans la possibilité d'utiliser une feuille du matériau sélectivement étanche, par exemple du TYVEK^{®}, dont l'utilisation avec une stérilisation fluidique à haute température, notamment pour des boîtes 2 de grande taille demande un certain nombre de précautions.

En effet, ceci est particulièrement le cas des boîtes pour lesquelles le rapport entre le volume libre intérieur (en litres) et la surface de scellage de l'opercule 18 sur la boîte 2 (en dm²) est supérieur à 2, et plus particulièrement encore pour celles dont le rapport est supérieur à 3.

L'utilisation d'un matériau sélectivement étanche pour obturer des emballages de dimension telle que décrite précédemment est donc possible, avec des stérilisations à haute température et ce dans des conditions industriellement et économiquement acceptables.

## Revendications

1. Opercule (18) pour emballage de produits stérilisés ou destinés à être stérilisés comprenant au moins une feuille d'un matériau sélectivement étanche, dont le seuil de coupure de l'extérieur vers l'intérieur de l'emballage arrête les particules contaminantes et permet le passage d'un fluide stérilisant thermique à des températures proches des températures de déformation de l'opercule, ledit matériau sélectivement étanche étant susceptible de se déformer dans le plan de la feuille auxdites températures, **caractérisé en ce qu'**il comprend un moyen de compensation planaire de la feuille de matériau sélectivement étanche, lorsque ledit opercule (18) est en contact avec le fluide stérilisant thermique auxdites températures, ledit moyen de compensation étant du type passif, et étant susceptible d'être obtenu par un prétraitement thermique du matériau sélectivement étanche, en le contractant définitivement de manière planaire.

2. Opercule (18) pour emballage de produits stérilisés ou destinés à être stérilisés comprenant au moins une feuille d'un matériau sélectivement étanche, dont le seuil de coupure de l'extérieur vers l'intérieur de l'emballage, arrête les particules contaminantes et permet le passage d'un fluide stérilisant thermique à des températures proches des températures de déformation de l'opercule, ledit matériau sélectivement étanche étant susceptible de se déformer dans le plan de la feuille auxdites températures, **caractérisé en ce qu'**il comprend un moyen de compensation planaire de la feuille de matériau sélectivement étanche, lorsque ledit opercule (18) est en contact avec le fluide stérilisant thermique auxdites températures, ledit moyen de compensation étant du type actif et consistant en une conformation en relief (18b), de la feuille du matériau sélectivement étanche, dont la surface développée excède la surface apparente de ladite feuille au point d'annuler, lors de son déploiement, la contrainte liée à la déformation planaire de ladite feuille.

3. Opercule selon la revendication 2, **caractérisé en ce que** la conformation en relief (18b) est du type plissage, repliage, bosselure, ondulation ou gaufrage.

4. Opercule (18) pour emballage de produits stérilisés ou destinés à être stérilisés comprenant au moins une feuille d'un matériau sélectivement étanche, dont le seuil de coupure de l'extérieur vers l'intérieur de l'emballage, arrête les particules contaminantes et permet le passage d'un fluide stérilisant thermique à des températures proches des températures de déformation de l'opercule, ledit matériau sélectivement étanche étant susceptible de se déformer dans le plan de la feuille auxdites températures, ledit emballage comportant au moins un organe de communication fluidique (16) entre l'intérieur et l'extérieur dudit emballage, **caractérisé en ce que**, ledit opercule comprend un moyen de compensation planaire de la feuille de matériau sélectivement étanche, lorsque ledit opercule (18) est en contact avec le fluide stérilisant thermique auxdites températures, ledit moyen de compensation étant du type actif, ledit opercule (18) comprenant un liséré (22) d'un matériau étanche ou non vis-à-vis du fluide stérilisant thermique, dont la bordure extérieure (22a) est susceptible d'être continûment liée de manière étanche au cadre de l'organe de communication fluidique (16), et dont la bordure intérieure (22b) est continûment liée de manière étanche à une feuille centrale (19) de matériau sélectivement étanche, ledit liséré (22) comprenant ledit moyen de compensation,
et **en ce que** le moyen de compensation consiste en une conformation en relief (22c), par exemple repliée, ondulée ou plissée sur elle-même, autour d'une ou plusieurs lignes continues circonscrivant la feuille centrale (19) en matériau sélectivement étanche, dont la surface développée excède la surface apparente dudit liseré, au point d'annuler, lors de son déploiement, la contrainte liée à la déformation planaire de ladite feuille.

5. Opercule selon la revendication 4, **caractérisé en ce que** le matériau étanche du liséré (22) est transparent vis-à-vis d'un rayonnement ultraviolet stérilisant ou décontaminant.

6. Opercule selon l'une des revendications 1 à 5, **caractérisé en ce que** le matériau sélectivement étanche est une nappe non tissée de fibres ou filaments, enchevêtrés et liés les uns aux autres.

7. Opercule selon la revendication 6, **caractérisé en ce que** les filtres ou filaments sont issus d'une matière plastique, par exemple PEHD ou autre polymère, liés par exemple par thermofusion.

8. Opercule (18) selon l'une des revendications 1 à 7, **caractérisé en ce qu'**il est associé à un écran (26) vis-à-vis d'une irradiation électronique et/ou d'un rayonnement lumineux, par exemple ultraviolet.

9. Opercule (18) selon la revendication 8, **caractérisé en ce que** l'écran (26) est rapporté sur lui par tout moyen, notamment par collage.

10. Emballage en matériau plastique, contenant une charge stérilisable ou stérilisée par mise en contact avec un fluide stérilisant thermique à des températures proches des températures de déformation du matériau plastique, ledit emballage comportant au moins un organe de communication fluidique (16) entre l'intérieur et l'extérieur dudit emballage consistant en une boîte (2) et au moins un cadre circonscrivant une ouverture, **caractérisé en ce qu'**il comprend un opercule (18) selon l'une des revendications 1 à 9, ledit opercule (18) obturant l'ouverture, la bordure périphérique (18a, 22a) dudit opercule (18) étant continûment liée de manière étanche audit cadre.

11. Emballage selon la revendication 10, **caractérisé en ce que** la liaison entre la bordure périphérique (18a) de l'opercule (18) et le cadre de l'organe de communication fluidique (16) est mécaniquement résistante vis-à-vis de la contrainte du fait de déformation planaire de la feuille du matériau sélectivement étanche constituant l'opercule (18).

12. Emballage selon la revendication 11, **caractérisé en ce que** la paroi (2a) de l'emballage est mécaniquement résistante vis-à-vis de la contrainte du fait de la déformation planaire de la feuille du matériau sélectivement étanche constituant l'opercule (18).

13. Emballage selon l'une des revendications 10 à 12, **caractérisé en ce qu'**il comporte un écran (26) destiné à être déposé sur des produits (10) à l'intérieur de la boîte (2) préalablement au scellage de celle-ci.

## Claims

1. Film (18) for packaging sterile products or products intended to be sterilized comprising at least one sheet of a selectively impervious material of which the cutoff threshold from the outside to the inside of the packaging stops contaminating particles and allows the passage of a thermal sterilizing fluid at temperatures close to the temperatures at which the film deforms, the said selectively impervious material being capable of deforming in the plane of the sheet at said temperatures, **characterized in that** it comprises a planar compensation means for the sheet of selectively impervious material when the said film (18) is in contact with the thermal sterilizing fluid at said temperatures, the said compensation means being of the passive type and being capable of being obtained by thermal pretreatment of the selectively impervious material, definitively shrinking it in a planar way.

2. Film (18) for packaging sterile products or products intended to be sterilized comprising at least one sheet of a selectively impervious material of which the cutoff threshold from the outside to the inside of the packaging stops contaminating particles and allows the passage of a thermal sterilizing fluid at temperatures close to the temperatures at which the film deforms, the said selectively impervious material being capable of deforming in the plane of the sheet at said temperatures, **characterized in that** it comprises a planar compensation means for the sheet of selectively impervious material when the said film (18) is in contact with the thermal sterilizing fluid at said temperatures, the said compensation means being of the active type and consisting in a shaping as a relief (18b) of the sheet of selectively impervious material, the developed surface area of which exceeds the visible surface area of the said sheet to such a point that, as it deploys, it cancels the stress associated with the planar deformation of the said sheet.

3. Film according to Claim 2, **characterized in that** the shaping as a relief (18b) is of the pleating, folding, embossing, corrugating or goffering type.

4. Film (18) for packaging sterile products or products intended to be sterilized comprising at least one sheet of a selectively impervious material of which the cutoff threshold from the outside to the inside of the packaging stops contaminating particles and allows the passage of a thermal sterilizing fluid at temperatures close to the temperatures at which the film deforms, the said selectively impervious material being capable of deforming in the plane of the sheet at said temperatures, said packaging comprising at least one member (16) for fluidic communication between the inside and the outside of the said packaging, **characterized in that** said film comprises a planar compensation means for the sheet of selectively impervious material when the said film (18) is in contact with the thermal sterilizing fluid at said temperatures, the said compensation means being of the active type, said film (18) comprising an edging (22) of a material which may or may not be impervious to the thermal sterilizing fluid, the outer border (22a) of which is capable of being continuously connected in sealed manner to the surround of the fluidic communication member (16), and the inner border (22b) of which is continuously connected in sealed manner to a central sheet (19) of selectively impervious material, the said edging (22) comprising the said compensation means, and **in that** the compensation means consists in a shaping as a relief (22c), for example folded, corrugated or pleated on itself, about one or more continuous lines circumscribing the central sheet (19) of selectively impervious material, the developed surface area of which exceeds the visible surface area of the said edging to such a point that, as it deploys, it cancels the stress associated with the planar deformation of the said sheet.

5. Film according to Claim 4, **characterized in that** the impervious material of the edging (22) is transparent to sterilizing or decontaminating ultraviolet radiation.

6. Film according to one of Claims 1 to 5, **characterized in that** the selectively impervious material is a nonwoven web of fibres or filaments which are entangled and joined together.

7. Film according to Claim 6, **characterized in that** the filters or filaments are from a plastics material, for example HDPE or some other polymer, connected, for example, by thermofusion.

8. Film (18) according to one of claims 1 to 7, **characterized in that** it is associated with a screen (26) against electron irradiation and/or light radiation, for example ultraviolet radiation.

9. Film (18) according to Claim 8, **characterized in that** the screen (26) is attached thereto by any means, in particular by bonding.

10. Packaging made of plastic, containing content which can be or has been sterilized by contact with a thermal sterilizing fluid at temperatures close to the temperatures at which the plastic deforms, said packaging comprising at least one member (16) for fluidic communication between the inside and the outside of the said packaging consisting in a tub (2) and at least one surround circumscribing an opening, **characterized in that** it comprises a film (18) according to one of claims 1 to 9, said film (18) closing the opening, the peripheral border (18a, 22a) of said film (18) being constantly connected in sealed fashion to said surround.

11. Packaging according to claim 10, **characterized in that** the connection between the peripheral border (18a) of the film (18) and the surround of the fluidic communication member (16) is mechanically able to withstand the stress resulting from the planar deformation of the sheet of selectively impervious material of which the film (18) is made.

12. Packaging according to claim 11, **characterized in that** the wall (2a) of the packaging is mechanically able to withstand the stress resulting from the planar deformation of the sheet of selectively impervious material of which the film (18) is made.

13. Packaging according to one of Claims 10 to 12, **characterized in that** it comprises a screen (26) intended to be deposited onto products (10) inside the tub (2) prior to the sealing of the latter.

## Patentansprüche

1. Film (18) zum Verpacken von sterilen Produkten oder Produkten, die sterilisiert werden sollen, mit zumindest einem Blatt aus einem selektiv undurchlässigen Material, dessen Ausschlussgrenze vom Äußerem ins Innere der Verpackung kontaminierende Partikel stoppt und ein Eindringen eines sterilisierenden thermischen Fluids mit Temperaturen nahe an Temperaturen der Deformation des Films ermöglicht, wobei das selektiv undurchlässige Material sich in der Ebene des Blattes bei diesen Temperaturen deformieren kann, **dadurch gekennzeichnet, dass** es ein ebenes Kompensationsmittel für das Blatt aus selektiv undurchlässigem Material aufweist, wenn der Film (18) mit dem sterilisierenden thermischen Fluid mit diesen Temperaturen in Kontakt ist, wobei die Kompensationsmittel von einem passiven Typus sind und welche durch eine thermische Vorbehandlung des selektiv undurchlässigen Materials erhalten werden können, wobei diese das Material endgültig auf eine ebene Art und Weise zusammenziehen.

2. Film (18) zum Verpacken von sterilen Produkten, oder Produkten, die sterilisiert werden sollen, mit zumindest einem Blatt aus einem selektiv undurchlässigen Material, dessen Ausschlussgrenze vom Äußeren zum Inneren der Packung kontaminierende Partikel stoppt und das Eindringen eines sterilisierenden thermischen Fluids bei Temperaturen nahe an Temperaturen der Deformation des Films ermöglicht, wobei das selektiv undurchlässige Material sich in der Ebene des Blattes bei diesen Temperaturen deformieren kann, **dadurch gekennzeichnet, dass** es ein ebenes Kompensationsmittel für das Blatt aus selektiv undurchlässigem Material aufweist, wenn der Film (18) mit dem sterilisierenden thermischen Fluid mit den besagten Temperaturen in Kontakt kommt, wobei die Kompensationsmittel aktiv sind und aus einer Gestaltung eines Reliefs (18b) des Blattes aus selektiv undurchlässigem Material bestehen, dessen entwickelte Oberfläche die sichtbare Oberfläche des Blattes bis zu einem Punkt überschreitet, dass, wenn es sich entfaltet, die Spannung, die mit der ebenen Deformation des Blattes assoziiert ist, aufgehoben wird.

3. Film nach Anspruch 2, **dadurch gekennzeichnet, dass** die Gestaltung des Reliefs (18b) nach Art einer Faltenbildung, einer Zusammenfaltung, Prägung, Welligkeit oder als Dessin vorgenommen ist.

4. Film (18) zum Verpacken von sterilen Produkten oder Produkten, die sterilisiert werden sollen, mit zumindest einem Blatt aus selektiv undurchlässigem Material, dessen Ausschlussgrenze vom Äußeren ins Innere der Verpackung kontaminierende Partikel aufhält und ein Eindringen eines sterilisierenden thermischen Fluids mit Temperaturen nahe bei der Temperatur der Deformation des Filmes ermöglicht, wobei das selektiv undurchlässige Material sich in der Ebene des Blattes bei diesen Temperaturen verformen kann, und wobei die Verpackung zumindest ein Element zur Fluid-Kommunikation (16) zwischen dem inneren und dem Äußeren der Verpackung aufweist, **dadurch gekennzeichnet, dass** der Film ein Mittel zur ebenen Kompensation des Blattes aus selektiv undurchlässigem Material aufweist, wenn der Film (18) mit dem sterilisierenden thermischen Fluid mit diesen Temperaturen in Kontakt ist, wobei die Kompensationsmittel vom aktiven Typus sind, und wobei der Film (18) e:ne Einfassung (22) aus einem gegenüber dem thermischen sterilisierenden Fluid undurchlässigen oder nicht-undurchlässigen Material aufweist, deren äußerer Rand (22a) fortlaufend dichtend mit dem Rahmen des Elements zur Fluid-Kommunikation (16) verbunden werden kann, und deren innerer Rand (22b) fortlaufend dichtend mit einem mittleren Blatt (19) aus einem selektiv undurchlässigen Material verbunden ist, wobei die Einfassung (22) die Kompensationsmittel aufweist, und **dadurch gekennzeichnet, dass** die Kompensationsmittel aus einer Gestaltung eines Reliefs (22c) bestehen, bspw. einem zusammengefalteten, gewellten oder einem plissierten Relief um eine oder mehrere kontinuierliche Anlagen herum, welche das mittlere Blatt (19) aus selektiv undurchlässigem Material begrenzen, dessen entwickelte Oberfläche die sichtbare Oberfläche der Einfassung bis zu einem Punkt überragt, dass bei der Entwicklung die Spannung, die mit der ebenen Deformation des Blattes assoziiert ist, aufgehoben wird,

5. Film nach Anspruch 4, **dadurch gekennzeichnet, dass** das dichte Material der Einfassung (22) gegenüber einer sterilisierenden oder dekontaminierenden Ultraviolettstrahlung durchlässig ist.

6. Film nach einem der Ansprüche 1 bis 5, **dadurch gekennzeichnet, dass** das selektiv undurchlässige Material ein nicht-gewebtes Flies aus Fasern oder Filamenten ist, die unlösbar miteinander verflochten und miteinander verbunden sind.

7. Film nach Anspruch 6, **dadurch gekennzeichnet, dass** die Fasern oder Filamente aus einem Kunststoffmaterial bestehen, bspw. aus PEHD oder einem anderen Polymer, und bspw. durch Thermofusion miteinander verbunden sind.

8. Film (18) nach einem der Ansprüche 1 bis 7, **dadurch gekennzeichnet, dass** er mi: einem Schirm (26) gegen elektronische und/oder eine Lichtstrahlung, bspw. ultraviolettes Licht, verbunden ist.

9. Film (18) nach Anspruch 8, **dadurch gekennzeichnet, dass** der Schirm (26) auf irgendeine Art und Weise darauf angebracht ist, insbesondere durch Aufkleben.

10. Verpackung aus Kunststoffmaterial, welches einen Inhalt enthält, der durch In-Kontakt-Bringen mit einem thermischen sterilisierenden Fluid bei Temperaturen nahe den Temperaturen der Deformation des Kunststoffmaterials sterilisiert wurde oder sterilisiert werden kann, wobei die Verpackung zumindest ein Element zur Fluid-Kommunikation (16) zwischen dem Inneren und dem Äußeren der Verpackung aufweist, welches aus einem Gehäuse (2) und zumindest einem eine Öffnung begrenzenden Rahmen besteht, **dadurch gekennzeichnet, dass** sie einen Film (18) gemäß einem der Ansprüche 1 bis 9 aufweist, wobei der Film (18) die Öffnung verdeckt, und der äußere Rand (18a, 22a) des Films (18) fortlaufend auf eine dichtende Art und Weise an dem Rahmen anliegt.

11. Verpackung nach Anspruch 10, **dadurch gekennzeichnet, dass** die Verbindung zwischen umgebendem Rand (18a) des Films (18) und des Rahmens des Elements zur Fluid-Kommunikation (16) der Spannung mechanisch widerstehen kann, welche durch die ebene Deformation des Blattes aus selektiv dichtem Material, aus welchem der Film (18) besteht, hervorgerufen wird.

12. Verpackung nach Anspruch 11, **dadurch gekennzeichnet, dass** die Wand (2a) der Verpackung einer Spannung mechanisch widersteht, die durch die ebene Deformation des Blattes aus selektiv dichtem Material, aus welchem der Film (18) besteht, hervorgerufen wird.

13. Verpackung nach einem der Ansprüche 10 bis 12, **dadurch gekennzeichnet, dass** sie einen Schirm (26) aufweist, der dazu vorgesehen ist, zwischen den Produkten (10) und dem Inneren des Gehäuses (2) angebracht zu werden, und zwar vor dessen Abdichtung.
